# FASCICULE DE BREVET EUROPEEN

(11) **EP 2 989 128 B1**
(45) Date de publication et mention de la délivrance du brevet: **19.07.2023**
(21) Numéro de dépôt: 14727589.5
(22) Date de dépôt: 23.04.2014
(51) Int. Cl.: A61P 27/02, A61P 43/00, A61P 7/00, A61P 19/00, A61K 31/738, A61L 27/26, A61L 27/50, A61L 27/52, A61L 27/54, C08B 37/02, C08B 37/08, C08J 3/075, C08J 3/24, C12N 5/00, C08L 5/00, C08L 5/02, C08L 5/08

(54) **HYDROGEL A BASE DE POLYSACCHARIDES NATIFS ET/OU FONCTIONNALISES, PHOSPHATES CO-RETICULES**
CO-VERNETZTES PHOSPHATIERTES NATIVES UND/ODER FUNKTIONALISIERTES HYDROGEL AUF POLYSACCHARIDBASIS
CO-CROSSLINKED PHOSPHATED NATIVE AND/OR FUNCTIONALIZED POLYSACCHARIDE-BASED HYDROGEL

(30) Priorité: 24.04.2013 FR 1353719
(43) Date de publication de la demande: 02.03.2016
(73) Titulaire: Eymeri, Manuel, 87000 Limoges (FR)
(72) Inventeur: DAHRI CORREIA, Latifa, F-38080 l'Isle d'Abeau (FR)
(74) Mandataire: Schmidt, Martin Peter
(86) Numéro de dépôt international: PCT/FR2014/050984
(87) Numéro de publication internationale: WO 2014/174213

(56) Documents cités:
- WO-A1-01/76652
- WO-A1-2009/047346
- DULONG V ET AL: "Hyaluronan-based hydrogels particles prepared by crosslinking with trisodium trimetaphosphate. Synthesis and characterization", CARBOHYDRATE POLYMERS, APPLIED SCIENCE PUBLISHERS, LTD. BARKING, GB, vol. 57, no. 1, 12 août 2004 (2004-08-12), pages 1-6, XP004522735, ISSN: 0144-8617, DOI: 10.1016/J.CARBPOL.2003.12.006

## Description

### Domaine technique de l'invention

L'invention concerne le domaine des hydrogels à base d'acide hyaluronique. En particulier elle concerne les hydrogels phosphorylés obtenus par co-réticulation d'acide hyaluronique avec le dextrane ainsi que son procédé de préparation. L'invention concerne également l'utilisation de cet hydrogel pour l'encapsulation et la libération prolongée de principes actifs ainsi que de cellules pour une utilisation en médecine régénérative humaine et vétérinaire.

### Etat de la technique

Le hyaluronane ou l'acide hyaluronique (CAS n° 9004-61-9, abrégé aussi ici HA) fait partie de la famille des glycosaminoglycanes. C'est un polymère de disaccharides, plus précisément un glycosaminoglycane linéaire non sulfaté composé d'unités répétitives de D-acide glucuronique et de N-acetyl-D-glucosamine liés entre eux par des liaisons glycosidiques alternées beta-1,4 et beta-1,3 (voir la publication de Tammi et al. « Hyaluronan metabolism in skin » paru dans la revue Progress in Histochemistry & Cytochemistry, 29 (2) : 1-81, 1994)).

L'acide hyaluronique se trouve dans de nombreux tissus des mammifères, et plus précisément dans la matrice extracellulaire de tissus. Grâce à sa capacité de lier des molécules d'eau, il a comme rôle principal l'hydratation des tissus. Si sa masse moléculaire dépasse environ 2 MDa et sa fraction massique environ 0,1%, il forme un hydrogel ; en fonction de sa masse moléculaire et de sa fraction massique il se trouve sous cette forme dans certain tissus, par exemple dans les articulations, et en particulier dans le liquide synovial. Il joue également d'autres rôles, par exemple dans la régulation de phénomènes biologiques comme la migration des cellules, la différentiation des tissus, l'angiogenèse et la régulation des cellules immunitaires. Ainsi, l'état de la technique attribue des fonctions assez différentes à cette molécule en fonction de sa masse moléculaire.

L'acide hyaluronique est dégradé dans le corps par un groupe d'enzymes appelés hyaluronidases (voir l'article « The magic glue hyaluronan and its eraser hyaluronidase : A biological overview » par K.S.Girish et K.Kemparaju, paru dans Life Sciences 80 (2007), p. 1921-1943) ; d'autres enzymes participent à ce processus.

Les hydrogels à base d'acide hyaluronique sont connus et utilisés dans de nombreuses applications médicales, notamment sous forme injectable dans la reconstruction cutanée, en chirurgie esthétique et en dermatologie, ou encore en orthopédie pour la reconstruction cartilagineuse et ostéoarticulaire (par exemple pour la viscosupplémentation du genou). Ils sont également utilisés en support de culture cellulaire pour la recherche et les thérapies cellulaires, ou en chimiothérapie.

Selon le mode de préparation et leurs utilisations, les hydrogels injectables à base d'acide hyaluronique peuvent être classés en trois catégories distinctes :
- les hydrogels de type « conductif », composé soit d'acide hyaluronique (AH) réticulé seul ou en combinaison avec un autre polymère, servant pour le comblement de rides en chirurgie esthétique ou en viscosupplémentation (restauration de l'homéostasie) ;
- les hydrogels de type « inductif » composé d'acide hyaluronique réticulé, contenant des facteurs de croissances et/ ou d'autres molécules dites bioactives pour le contrôle de leur délivrance in situ ;
- les hydrogels pour l'implantation cellulaire constitués quant à eux de plusieurs composantes : polymères, protéines et cellules.

Pour une utilisation en médecine humaine ou vétérinaire, un hydrogel doit répondre à de nombreux critères (cf. M.N.Collins et C.Birkinshaw, « Hyaluronic acid base scaffolds for tissue engineering - A review», Carbohydrate Polymers 92 (2013), 1262-1279 ; 2013 ; G.D.Prestwich, « Hyaluronic acid-based clinical biomaterials derived for cell and molécule delivery in regenerative medicine », J. Controlled Release 155 (2011), 193-199). Par exemple, l'hydrogel doit être biodégradable, biocompatible et résorbable ; et doit être facilement utilisable dans des conditions physiologiques.

On entend habituellement par « biocompatibilité » la capacité d'un matériau non vivant appelé à interagir avec les systèmes biologiques à remplir une fonction spécifique avec une réponse appropriée à l'hôte.

Le collagène, une famille de glycoprotéines, a été longuement utilisé comme produit de comblement résorbable injectable. Mais le collagène présente certains inconvénients qui sont : le risque d'apparitions de réactions allergiques et sa rapide dégradation in vivo.

Ce sont les raisons pour lesquelles il est remplacé par des sels d'acide hyaluronique qui ne présentent aucune réaction allergique et sont biocompatibles quel que soit leur origine et mode de préparation (animale, biotechnologie).

Aujourd'hui, les hydrogels d'acide hyaluronique, en particulier ses sels et/ou dérivés, ont pris une part non négligeable dans le domaine esthétique, particulièrement pour le comblement des rides et des défauts cutanés.

Les acides hyaluroniques et leurs sels peuvent être utilisés seuls pour la fabrication d'hydrogel, mais peuvent également être associés à d'autres polymères (collagène, gélatine, chitosane, sulfate de chondroïtine, ...) pour bénéficier d'une synergie des différents composés, et mimer le plus fidèlement la matrice extracellulaire.

L'acide hyaluronique peut être modifié chimiquement grâce aux fonctions carboxyliques, hydroxyles et acétamides, présentes le long de la chaîne. Ces groupements peuvent être utilisés d'une part pour sa réticulation, et d'autre part pour sa fonctionnalisation afin de lui apporter de nouvelles propriétés biologiques ou de lui améliorer ses propriétés biologiques et/ou physico-chimiques intrinsèques. La réticulation implique la création de liaisons entre deux chaînes de polysaccharides et/ou entre deux segments de la même chaîne. Elle résulte en une augmentation de la viscosité et/ou de la masse moléculaire, et entraîne la modification d'autres propriétés physiques et physico-chimiques.

Ainsi, les possibilités de préparation d'hydrogels à partir d'acide hyaluronique sont nombreuses.

Le document FR 2 918 377 décrit un gel cohésif co-réticulé comprenant au moins un premier gel de polysaccharide fortement réticulé, et au moins un second gel de polysaccharide faiblement réticulé, le premier gel étant lié par liaisons de covalence au second gel. Le gel fortement réticulé et le gel faiblement réticulé sont de préférence à base de hyaluronate de sodium ; l'agent de réticulation est le 1,4-butanediol diglycidyl éther (BDDE).

Le document US 6,586,493 décrit un hydrogel obtenu à partir d'un mélange comprenant un polysaccharide partiellement insaturé, substitué par un groupement de réticulation, et un acide hyaluronique non-angiogénique. La réticulation du mélange est réalisée par addition d'un initiateur de polymérisation radicalaire, en particulier un acétophénone (photo-initiateurs). Ce procédé est très désavantageux car de nombreux produits de dégradation issus de la polymérisation radicalaire peuvent être piégés dans l'hydrogel une fois formé. WO 2009/047346 décrit des structures d'échafaudage poreuses obtenues par plusieurs autres combinaisons de polysaccharides.

La présente invention vise à présenter de nouveaux hydrogels à base de polysaccharides, présentant une bonne résistance à la dégradation accrue, une absence de cytotoxicité, qui sont injectables, et biocompatibles pour un usage en chirurgie reconstruction cartilagineuse et ostéoarticulaire et en médecine régénérative humaine & vétérinaire.

### Objets de l'invention

La présente invention a pour objet un hydrogel phosphorylé co-réticulé de polysaccharides, à base de (i) dextrane et (ii) d'acide hyaluronique et/ou d'un de ses sels, éventuellement fonctionnalisé, dans lequel hydrogel ledit dextrane et ledit acide hyaluronique (et/ou son sel, éventuellement fonctionnalisé) sont liés entre eux par des liaisons de covalence phosphodiesters et/ou polyphosphodiesters.

Avantageusement, l'acide hyaluronique, ou son sel, est un acide hyaluronique (et/ou son sel) fonctionnalisé phosphoryle par le trimétaphosphate de sodium.

Dans un mode de réalisation particulier selon l'invention, le taux de concentration en phosphate de l'acide hyaluronique (et/ou de son sel) fonctionnalisé par le trimétaphosphate de sodium est compris entre 0,01 et 4 mEq/g, plus préférentiellement entre 0,1 et 2 mEq/g.

Dans un autre mode particulier de réalisation selon l'invention, le taux de concentration en phosphate de l'hydrogel est compris entre 0,1 et 3 mEq/g, plus préférentiellement entre 0,1 et 2 mEq/g.

La présente invention a aussi pour objet un procédé de préparation d'un hydrogel phosphorylé co-réticulé de polysaccharides, à partir de dextrane et d'acide hyaluronique et/ou un de ses sels, éventuellement fonctionnalisé, dans lequel procédé :
a) on approvisionne une solution de dextrane ;
b) on active au moins un groupe hydroxyle du dextrane en ajoutant une solution d'hydroxyde alcalin, par exemple de l'hydroxyde de sodium, à ladite solution de dextrane pour obtenir une solution de dextrane activé ;
c) on ajoute à ladite solution de dextrane activé du trimétaphosphate de sodium ;
d) on ajoute à la solution obtenue à l'étape c) de l'acide hyaluronique et/ou un de ses sels, éventuellement fonctionnalisé.

Avantageusement, les étapes c) et d) du procédé sont réalisées concomitamment, le trimétaphosphate de sodium et l'acide hyaluronique et/ou un de ses sels, éventuellement fonctionnalisé, étant ajoutés concomitamment dans la solution obtenue à l'étape b) dudit procédé.

Les étapes a) à d) dudit procédé sont réalisées à une température comprise entre 18 et 25°C.

La concentration en hydroxyde alcalin ajouté à l'étape b) est comprise entre 0,5M et 5M, de préférence entre 0,5M et 1M.

Dans un mode de réalisation particulier du procédé, l'acide hyaluronique et/ou un de ses sels ajouté à l'étape d) du procédé est un acide hyaluronique fonctionnalisé par le trimétaphosphate de sodium.

Avantageusement, l'acide hyaluronique et/ou un de ses sels, éventuellement fonctionnalisé, est ajouté sous la forme d'une poudre.

La présente invention a également pour objet un hydrogel selon l'invention, pour la préparation d'un médicament, destiné à être implanté seul ou associé à des cellules (éventuellement cellules souches) pour la thérapie cellulaire, ou destiné à être utilisé sous forme humide pour application topique, ou destiné à délivrer des molécules actives et principes actifs, et plus particulièrement des facteurs de croissances, de préférence les facteurs de croissance sélectionnés parmi les : facteurs de croissance des fibroblastes (FGFs), les facteurs de croissance dérivés des plaquettes (PDGFs), les facteurs de croissance de transformation (TGFs), les facteurs de croissance de l'endothélium vasculaire (vEGF), les facteurs de croissance ostéoinducteurs (BMPs) ; ou destiné à être utilisé en médecine régénérative, et plus particulièrement en médecine régénérative humaine et/ou vétérinaire.

Un dernier objet de l'invention est l'utilisation d'un hydrogel selon l'invention pour l'ostéoconductivité et/ou l'ostéoinductivité, i.e. pour la régénération osseuse.

### Description des figures

Les figures 1 à 8 illustrent certains aspects de l'invention.
La figure 1 est un spectre infrarouge (IR) suivant l'évolution du profil infrarouge d'un mélange réactionnel du procédé selon l'invention (exemple 1) sur 24 heures à partir de l'ajout concomitant d'acide hyaluronique et de trimétaphosphate de sodium TMP (courbe a : 22 minutes après ajout ; courbe b : 32 minutes après ajout ; courbe c : 42 minutes après ajout ; courbe d : 52 minutes après ajout ; courbe e : 120 minutes après ajout ; courbe f : gel obtenu après 24h à 25°C). L'abscisse représente le nombre d'ondes exprimé en cm⁻¹ ; l'ordonnée représente la transmittance (en %, sans dimension).
La figure 2 est un graphique représentant l'influence de la concentration en hydroxyde alcalin et de la concentration en trimétaphosphate de sodium sur la réticulation du dextrane.
La figure 3 est un spectre RMN ³¹P de l'hydrogel co-réticulé préparé selon l'invention après 24 heures.
La figure 4 représente deux spectres RMN ³¹P du trimétaphosphate de sodium à 25°C en présence de NaOH à 0,5M et en présence de NaOH à 3M.
La figure 5 est un spectre RMN ³¹P permettant de déterminer la quantité de phosphate d'un échantillon de gel déshydraté par hydrolyse en milieu fortement acide (HCl 4N).
La figure 6 est un graphique illustrant l'impact des acides hyaluroniques fonctionnalisés (HH) par le trimétaphosphate de sodium sur la prolifération des fibroblastes de derme humain en fonction de leur concentration et de leur degré de fonctionnalisation (lu par spectrophotométrie à 450 nm).
La figure 7 est un graphique comparant le pourcentage massique de facteurs de croissance de fibroblastes non absorbés par les hydrogels selon l'invention après leurs mises en contact avec les hydrogels pendant 24 heures dans une solution de NaCl.
La figure 8 est un graphique illustrant l'impact des hydrogels selon l'invention sur la prolifération cellulaire, et en particulier sur la prolifération des cellules souches appelées cellules « progéniteurs endothéliaux circulants ».

### Description détaillée de l'invention

### 1. Définitions

Dans l'exposé de la présente invention, le terme « acide hyaluronique » comprend les sels de l'acide hyaluronique.

Par « hydrogel phosphorylé » on entend un hydrogel dont la structure chimique comprend au moins une liaison covalente phosphodiester et/ou polyphosphodiester.

Par « agent de co-réticulation » (ou agent co-réticulant), on entend un agent permettant la formation des liaisons de covalence entre deux polysaccharides différents, plus particulièrement entre un acide hyaluronique, éventuellement fonctionnalisé, ou un de ses sels, et le dextrane ; l'agent de co-réticulation étant choisi parmi un agent de réticulation (ou agent réticulant) connu lui-même pour effectuer la réticulation de ces mêmes polysaccharides. Ainsi, dans le cadre de la présente invention, les termes « agent de co-réticulation » et « agent réticulant » se rapportent au même agent chimique (i.e. le trimétaphosphate de sodium).

Par « hydrogel co-réticulé de polysaccharides » on entend un hydrogel comprenant au moins deux polysaccharides différents liés entre eux par des liaisons de covalence.

Par « hydrogel phosphorylé co-réticulé de polysaccharides » on entend un hydrogel comprenant au moins deux polysaccharides différents liés entre eux par des liaisons de covalence phosphodiesters et/ou polyphosphodiesters telles que représentées ci-après (R₁ et R₂ représentent respectivement un premier et un deuxième polysaccharide substitué).

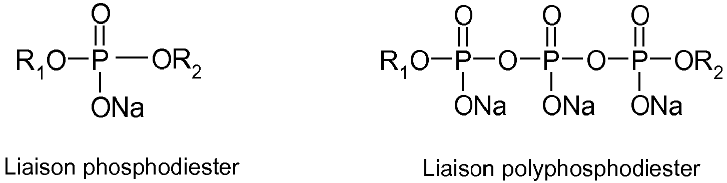

Par « acide hyaluronique fonctionnalisé » (abrégé aussi ici HH) on entend un acide hyaluronique (ou un de ses sels) substitué (au moins partiellement) par divers groupements organiques.

Dans la description détaillée de l'invention qui suit, les mécanismes réactionnels énoncés ne sont que des hypothèses et modèles heuristiques qui ne limitent pas la portée de l'invention.

### 2. Description détaillée

Dans le cadre de la présente invention, la demanderesse a mis en évidence qu'un acide hyaluronique, éventuellement fonctionnalisé, ou un de ses sels, en mélange avec un dextrane activé, utilisant le trimétaphosphate de sodium comme agent de réticulation, permet l'obtention d'un hydrogel phosphorylé co-réticulé présentant des propriétés biologiques améliorées. La demanderesse a démontré que les objectifs de l'invention sont atteints grâce à l'obtention d'un hydrogel phosphorylé co-réticulé de polysaccharides obtenus par un procédé spécifique et optimisé décrit plus en détail ci-après.

Selon l'invention, les acides hyaluroniques peuvent éventuellement être utilisés sous la forme d'un sel physiologiquement acceptable, et peuvent être fonctionnalisés.

Selon un premier aspect, la présente invention concerne un procédé de préparation d'un hydrogel phosphorylé co-réticulé de polysaccharides, ledit procédé comprenant les étapes suivantes :
a) On approvisionne une solution de dextrane ;
b) On active au moins un groupe hydroxyle du dextrane en ajoutant une solution d'hydroxyde alcalin (par exemple de l'hydroxyde de sodium NaOH) à ladite solution de dextrane ; cette étape conduit à la transformation dudit au moins un groupe hydroxyle en groupe alcoolate, et le dextrane ainsi transformé est appelé « dextrane activé », selon la réaction :

   DexOH + NaOH → DexONa
c) On ajoute à ladite solution de dextrane activé du trimétaphosphate de sodium. Cette étape conduit à la réticulation du dextrane activé par attaque nucléophile de l'oxygène du groupe alcoolate de dextrane sur un phosphore du triméthylphosphate, comme indiqué sur le schéma réactionnel 1.1) ci-après. Plusieurs réactions peuvent se produire au cours de la réticulation selon les schémas réactionnels ci-après (la flèche émanant du DexONa symbolise l'attaque nucléophile de l'oxygène de l'alcoolate de dextrane sur le phosphore du trimétaphosphate). 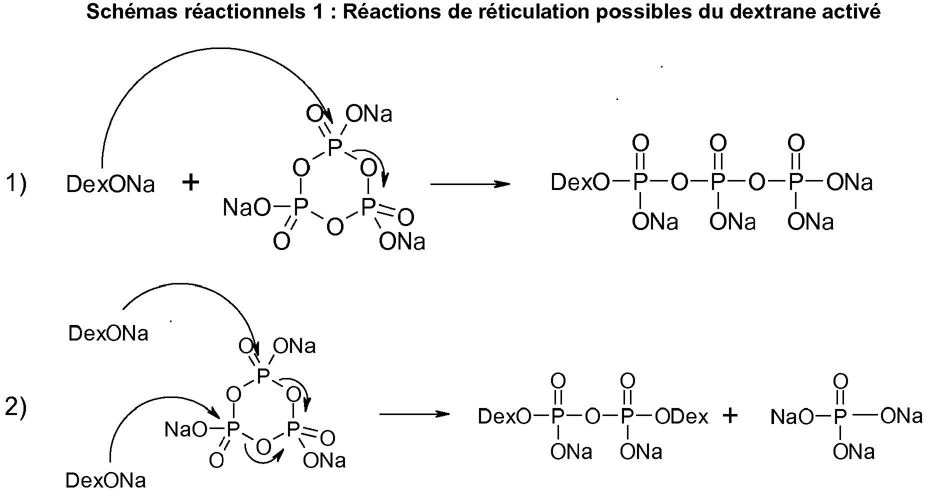 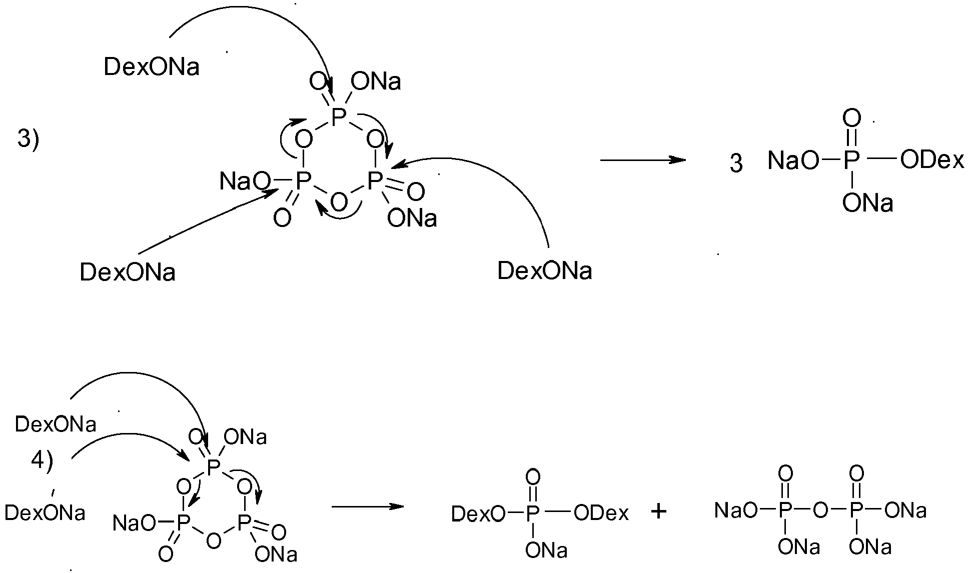
d) On ajoute à la solution obtenue à l'étape c) de l'acide hyaluronique (HA), éventuellement fonctionnalisé (HH), ou l'un de ses sels ; de préférence cet ajout se fait avec de la poudre et non pas avec une solution de l'acide hyaluronique (éventuellement fonctionnalisé, ou l'un de ses sels). Pendant l'ajout de l'acide hyaluronique (HA ou HH) et pendant la formation de l'hydrogel le milieu réactionnel est maintenu sous agitation. La température lors de cette étape est avantageusement comprise entre 18°C et 25°C.

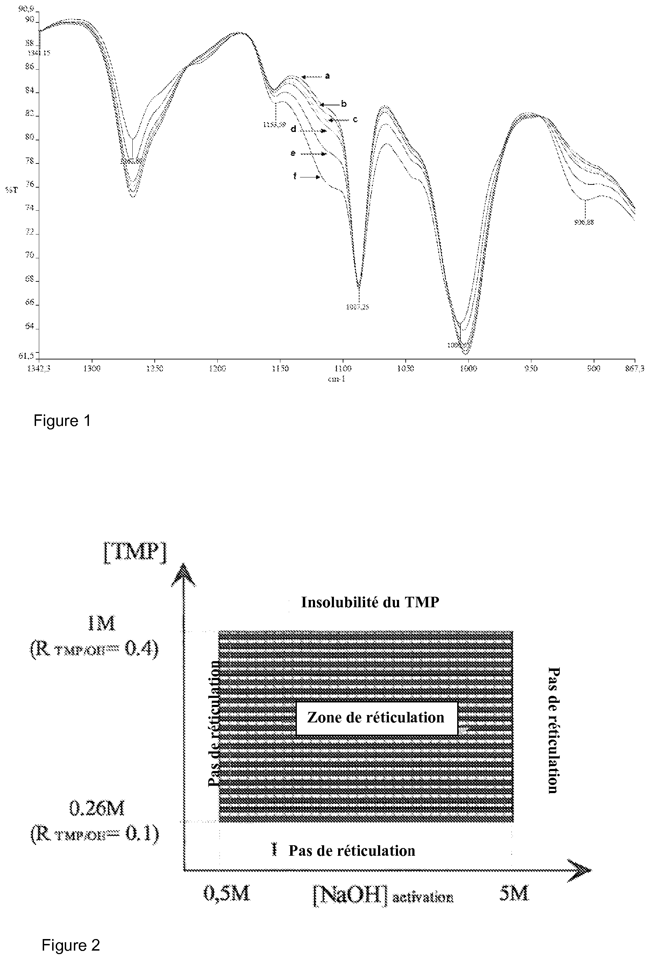

Cette étape d) conduit à la co-réticulation du dextrane activé avec l'acide hyaluronique, éventuellement fonctionnalisé, ou l'un de ses sels, pour former un hydrogel phosphorylé co-réticulé de polysaccharides, le mélange étant maintenu sous agitation jusqu'à obtention de l'hydrogel. Le temps de réaction est typiquement compris entre 10 et 40 minutes (de préférence entre 20 et 30 minutes) pour former l'hydrogel. Ensuite on laisse reposer l'hydrogel figé à température ambiante (typiquement 18 - 25°C) pour que la réaction puisse se terminer. L'analyse par spectroscopie Infrarouge à Transformation de Fourrier (FT-IR) met en évidence la présence d'esters phosphorique sur la chaîne du polysaccharide (cf. figure 1 et les explications données dans les exemples).

L'hydrogel phosphorylé selon l'invention comprend donc un premier polysaccharide (dextrane), et un deuxième polysaccharide (acide hyaluronique, éventuellement fonctionnalisé, ou un de ses sels), lesdits polysaccharides étant liés entre eux par des liaisons de covalence de type phosphodiesters et/ou polyphosphodiesters.

Le trimétaphosphate de sodium ou « TMP » (CAS n° 7785-84-4), de formule brute Na₃P₃O₉, est un composé non toxique pour l'homme (voir par exemple: K.Woo et P.A.Seib, Carbohydrate Polymers 1997 (33), 263-271), couramment utilisé dans l'industrie alimentaire. Le trimétaphosphate de sodium peut subir des attaques nucléophiles permettant ainsi l'ouverture du cycle de la TMP. Un mécanisme réactionnel décrivant l'attaque nucléophile d'un alcoolate de polysaccharide sur le trimétaphosphate de sodium a été proposé dans la littérature scientifique (voir l'article « High-resolution nuclear magnetic résonance spectroscopy studies of polysaccharides cross-linked by sodium trimétaphosphate : a proposal for the reaction mechanism » par S. Lack et al. Paru dans Carbohydrate Research 342 (2007) p. 943-953).

L'utilisation de trimétaphosphate de sodium en tant qu'agent réticulant et co-réticulant est essentielle dans le procédé selon l'invention, car il permet l'obtention de polysaccharides réticulés phosphatés insolubles dans l'eau. De plus, de par l'utilisation spécifique de cet agent chimique et de par l'effet synergique entre l'acide hyaluronique, éventuellement fonctionnalisé, et le dextrane, l'hydrogel phosphorylé co-réticulé de polysaccharides selon la présente invention présente une meilleure résistance à la dégradation enzymatique par les hyaluronidases (enzymes responsables de la dégradation de l'acide hyaluronique dans l'organisme) qu'un hydrogel d'acide hyaluronique simplement réticulé (i.e. un hydrogel d'acide hyaluronique réticulé sur lui-même, sans ajout d'un autre polysaccharide). Enfin, l'hydrogel selon l'invention présente une amélioration très nette sur la prolifération cellulaire.

Les conditions de préparation d'un hydrogel phosphorylé co-réticulé sont présentées ci-après.

Dans un premier temps, la préparation d'un hydrogel à base de dextrane réticulé, au moyen du trimétaphosphate de sodium, est réalisée. La réaction de réticulation du dextrane se déroule en deux étapes ; la première étape consiste en l'activation des groupes hydroxyles du dextrane les plus réactifs (i.e. les groupes hydroxyles en C₂ du dextrane) pour former un alcoolate de dextrane (appelé aussi ici dextrane activé), puis en une attaque nucléophile de l'alcoolate de dextrane sur le trimétaphosphate de sodium (cf. les schémas réactionnels ci-avant).

Durant la réaction de réticulation, certaines réactions secondaires sont observées. Il s'agit en particulier de l'hydrolyse basique des ponts phosphatés formés, solubilisant ainsi l'hydrogel (cf. schéma réactionnel n°2 ci-après). Une autre réaction secondaire est la dégradation basique du trimétaphosphate de sodium en tripolyphosphate de sodium (TPP) (cf. schéma réactionnel n°3 ci-après). Ces réactions secondaires ne sont pas souhaitables dans le cadre de la présente invention.

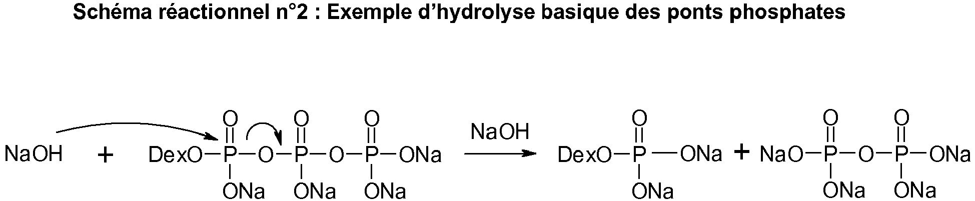

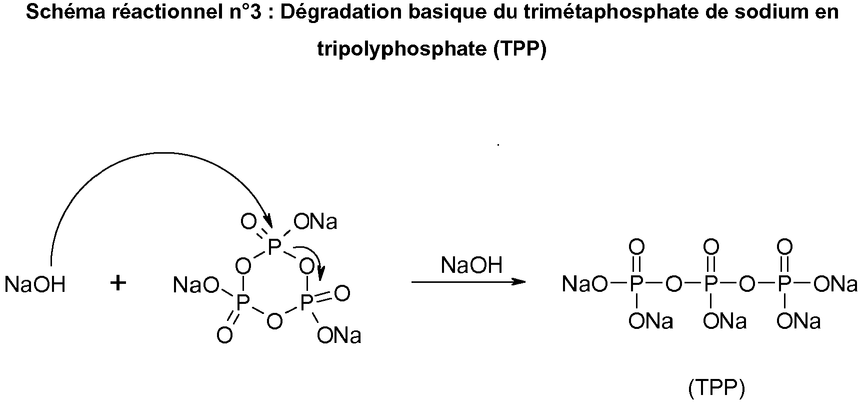

Afin de limiter ces réactions secondaires, l'inventeur a trouvé une plage optimale pour la concentration en hydroxyde alcalin et en trimétaphosphate de sodium TMP (cf. figure 2 : la concentration en hydroxyde alcalin indiquée sur la figure 2 correspond à la concentration de hydroxyde alcalin lors de la phase d'activation du dextrane ; la concentration en TMP indiquée correspond à la concentration en TMP dans le milieu réactionnel (dextrane + hydroxyde alcalin + TMP)). La concentration en hydroxyde est comprise entre 0,5 M et 5 M pour réaliser l'activation des groupes hydroxyles du dextrane. Dans un mode de réalisation préférée, la concentration en hydroxyde lors de l'étape dite d'activation est comprise entre 0,5 M et 1 M. De même, la concentration en TMP dans le mélange réactionnel est de préférence comprise entre 0,26 M et 1 M, ce qui correspond à un ratio [TMP]/[OH] compris entre 0,1 et 0,4. En effet, pour une concentration en hydroxyde supérieure à 5 M, l'hydrolyse basique des ponts phosphates est favorisée au détriment de la réticulation du dextrane. De plus, pour une concentration en TMP inférieure à 0,26 M, aucune réticulation n'est observée, et pour une concentration supérieure à 1 M le TMP devient insoluble dans l'eau.

Avantageusement, le procédé selon l'invention est réalisé à une température comprise entre 18 et 25°C. En effet, même si une augmentation de la température du milieu réactionnel a pour effet de réduire le temps de gélification de la solution, elle accélère l'hydrolyse des ponts phosphates formés. C'est pourquoi il est plus avantageux de réaliser le procédé à température ambiante afin de limiter l'hydrolyse des ponts phosphates formés. Au-dessous de 18°C, la réaction de réticulation devient très lente. Dans le procédé de préparation décrit, les différentes étapes du procédé sont réalisées dans un milieu basique dont la valeur du pH est sensiblement la même. Chacune des étapes est effectuée en milieu basique à un pH pouvant être compris entre 8 et 14, de préférence entre 8 et 10.

Dans un mode de réalisation très avantageux du procédé selon l'invention, l'acide hyaluronique, éventuellement fonctionnalisé ou un de ses sels, est ajouté dans le milieu réactionnel en même temps que le trimétaphosphate de sodium (et de manière séparée) ; les étapes c) et d) du procédé selon l'invention sont ainsi confondues. La demanderesse a pu observer que les meilleurs résultats de gélification ont été obtenus par cette méthode. En effet, pour des questions de stabilité de l'acide hyaluronique en milieu basique (ou de l'acide hyaluronique fonctionnalisé), il est avantageux de l'ajouter en même temps que le trimétaphosphate de sodium. Cet ajout de HA et/ou HH se fait avantageusement sous la forme de poudre, et il en est de même du trimétaphosphate de sodium ; cela augmente la réticulation et diminue l'importance des réactions secondaires par rapport à un ajout en solution. En tous les cas, qu'il soit fait sous la forme de poudre (préféré) ou sous la forme de solution (moins préféré), l'ajout simultané de HA et/ou HH, d'une part, et de trimétaphosphate de sodium d'autre part se fait avantageusement de manière spatialement séparée, i.e. l'ajout des poudres et ou des solutions séparées se fait à des endroits différents du mélange réactionnel.

De préférence, l'acide hyaluronique (HA et/ou HH) utilisé dans le procédé selon la présente invention présente une masse moléculaire compris entre 10 et 5 000 kDa, de préférence comprise entre 10 kDa et 1 200 kDa, et plus préférentiellement comprise entre 25 et 1200 kDa. Ce choix est motivé par l'utilisation de l'hydrogel selon l'invention comprenant un acide hyaluronique, éventuellement fonctionnalisé, phosphorylé de masse moléculaire pour ses effets biologiques :
- l'acide hyaluronique de faiendoble masse moléculaire, i.e. inférieur à 50 kDa, de préférence compris entre 25 et 50 kDa, et plus préférentiellement comprise entre 10 et 50 kDa, est préféré pour induire un effet angiogénique ;
- l'acide hyaluronique de masse moléculaire comprise entre 50 et 300 kDa est préféré pour la prolifération et la migration cellulaire ;
- l'acide hyaluronique de masse moléculaire supérieure à 800 kDa et jusqu'à 5 000 kDa est préféré pour son effet de maintien de l'hydratation des tissus.

De préférence, le dextrane utilisé, dans le procédé selon la présente invention présente une masse moléculaire comprise entre 10 kDa et 2 000 kDa, de préférence comprise entre 40 kDa et 500 kDa, également pour des raisons liées à l'utilisation de l'hydrogel selon l'invention (sachant que cette préférence est moins forte que pour ce qui concerne la masse moléculaire de HA et HH).

Dans un mode de réalisation particulièrement avantageux, l'acide hyaluronique ajouté à l'étape d) du procédé selon l'invention [cette étape d) étant possiblement exécutée en même temps que l'étape c)] est un acide hyaluronique fonctionnalisé par le trimétaphosphate de sodium. La synthèse de cet acide hyaluronique fonctionnalisé s'effectue en deux étapes. Dans une première étape dite d'activation, on ajoute une solution d'hydroxyde de sodium pour activer les groupes hydroxyles de l'acide hyaluronique. De préférence, la phase d'activation est réalisée à un pH basique inférieur à 8 (sinon une nouvelle réaction secondaire, à savoir l'ouverture spontanée de la molécule cyclique de trimétaphosphate, peut se produire). On réalise ensuite une étape dite de fonctionnalisation par ajout de trimétaphosphate de sodium dans la solution d'acide hyaluronique sous forme alcoolate.

Après l'étape d) du procédé selon l'invention, l'acide hyaluronique fonctionnalisé, phosphorylé est purifié et séché selon des techniques classiques de l'homme du métier, et notamment l'acide hyaluronique fonctionnalisé peut être précipité dans de l'éthanol (ou l'acétone) pour éliminer les traces résiduelles d'agent co-réticulant puis dialysé contre de l'eau osmosée jusqu'à ce que le pH et la conductimétrie des eaux de dialyse soient proches de celles de l'eau osmose (pH 5,6 = et conductimétrie < 30 µs/cm ). L'acide hyaluronique ainsi purifié peut être lyophilisé.

L'inventeur a constaté que la dégradation enzymatique de l'hydrogel selon l'invention par l'enzyme hyaluronase est plus rapide que la dégradation par l'enzyme dextranase, et que la dégradation avec chacun des deux enzymes est ralentie si l'hydrogel est fortement réticulé.

Pour l'application de l'hydrogel selon l'invention en tant que support pour la culture cellulaire (en particulier en recherche et en thérapie cellulaire), un aspect important est le degré de fonctionnalisation de l'acide hyaluronique par le phosphate. L'amélioration de la prolifération cellulaire n'est observée qu'à partir d'une teneur en phosphate de 0,01 mEq/g. La teneur est préférentiellement d'au moins 0,05 mEq/g, et encore plus préférentiellement d'au moins 0,1 mEq/g (une unité « mEq/g » appelée aussi « milli-équivalent/g » équivaut à une millimole par gramme de produit).

Le produit selon l'invention présente de nombreux avantages, et notamment un excellent compromis des propriétés suivantes : propriétés physico-chimiques, biocompatibilité, implantation, la résistance à la dégradation enzymatique, absence de cytotoxicité. Ce résultat est obtenu grâce à l'optimisation de la mise en oeuvre de la co-réticulation de l'acide hyaluronique avec le dextrane pour préparer un hydrogel à base de polysaccharides phosphorylé co-réticulé.

Nous indiquons ici à titre d'exemple quelques utilisations de l'hydrogel selon l'invention.

Pour l'utilisation de l'hydrogel selon l'invention en thérapie cellulaire, on prépare un hydrogel selon l'invention et incorpore les nutriments cellulaires (acides aminés, etc.) et facteurs de croissance appropriés. Ce produit peut être stocké sous forme lyophilisée et/ou congelée. Après réhydratation du produit lyophilisé l'hydrogel peut être utilisé sous forme topique (par exemple comme pansement) ou injectable.

Pour l'utilisation de l'hydrogel selon l'invention en tant que système de délivrance de principe actif, on prépare un hydrogel selon l'invention sous forme de poudre, on hydrate l'hydrogel (soit par du sérum physiologique soit par une quantité prédéterminée de principe(s) actif(s), par exemple un facteur de croissance et/ou un anti-inflammatoire et/ou un antibiotique, ou encore du plasma riche en plaquettes pour obtenir un hydrogel qui présente la consistance souhaitée (gel imbibé)), et on dépose le produit sur le site visé (par exemple sur une microfissure osseuse, une micro-déchirure ou déchirure d'un tendon, une déchirure musculaire).

D'autres buts, caractéristiques et avantages de l'invention sont maintenant illustrés par les exemples ci-après, sans toutefois apporter une quelconque limitation à l'invention.

### Exemple 1 : Préparation d'un hydrogel co-réticulé à partir d'un acide hyaluronique (HA) et de dextrane (procédé selon l'invention).

*i)* *Activation des groupes hydroxyles du dextrane par une solution de NaOH 0,5* M 0,9 grammes de dextrane T100 (CAS n° 9004-54-0 ; 100 000 g/mol) ont été mis en solution dans 50 ml d'eau, puis 5 ml d'hydroxyde de sodium NaOH 0,5 M ont été ajoutés à la solution. Le mélange a été maintenu sous agitation pendant 30 minutes à température ambiante.
*ii)* 0,1 gramme d'acide hyaluronique (500K, mol wt 400-600 kDa) (ratio HA/HA+dextrane = 10%) et 1,02 grammes de trimétaphosphate de sodium (utilisé entant qu'agent réticulant) ont été ajoutés simultanément (sous la forme de poudres, spatialement séparées) à la solution de l'étape a).
   Le mélange a été maintenu sous agitation à température ambiante jusqu'à gélification, puis le gel obtenu a été conservé pendant 5 heures à 25°C.
*iii) Lavage, purification et séchage de l'hydrogel*
   L'hydrogel a été broyé sur un tamis en inox et récupéré dans une solution d'acétone. Plusieurs lavages dans l'eau ont été effectués avec un suivi du pH, jusqu'à l'obtention d'un pH < 7 des eaux de lavage ; pour chaque étape de lavage réalisé, l'hydrogel a été filtré sur Büchner avec un filtre en acétate de cellulose. Un dernier lavage avec de l'acétone a été effectué, puis l'hydrogel a été séché à l'étude sous vide à 60°C.
*iv) Caractérisation chimique*

L'évolution de la réaction de co-réticulation par spectroscopie IR de l'acide hyaluronique (500K, mol wt 400-600 kDa) et du dextrane T100 est illustrée en figure 1. Les conditions expérimentales ont été les suivantes :
Spectromètre FT-IR Perkin Elmer Instrument « Spectrum One », analyses réalisées par sonde ATR (attenuated total reflection)
Les caractéristiques des bandes principales d'absorbance IR sont indiquées dans le tableau 1 ci-après.

**Tableau 1 : Caractéristiques des principales bandes d'absorbance IR**

| Nombre d'onde (cm⁻¹) | Groupement | Mode de vibration |
|---|---|---|
| 1260 | P=O (phosphonate) | Elongation |
| 1153 | -OH secondaire | Elongation |
| 1087 | -OH secondaire | Vibration de valence (vC-O) |
| 1006 | -OH primaire | Vibration de valence (vC-O) |
| 906 | P-OR (ester phosphorique) | Elongation |

Au cours de la réaction de co-réticulation, le suivi de la réaction par spectroscopie infrarouge met en évidence l'attaque nucléophile des alcoolates sur le trimétaphosphate de sodium, les bandes caractéristiques des alcools primaires (1006 cm⁻¹) diminuant au dépend des bandes caractéristiques des alcools secondaires (1153 cm⁻¹). Les bandes d'absorbance IR à 1260 cm⁻¹ et 906 cm⁻¹ mettent en évidence la formation d'ester phosphorique et de phosphonate (liaison phosphodiester).

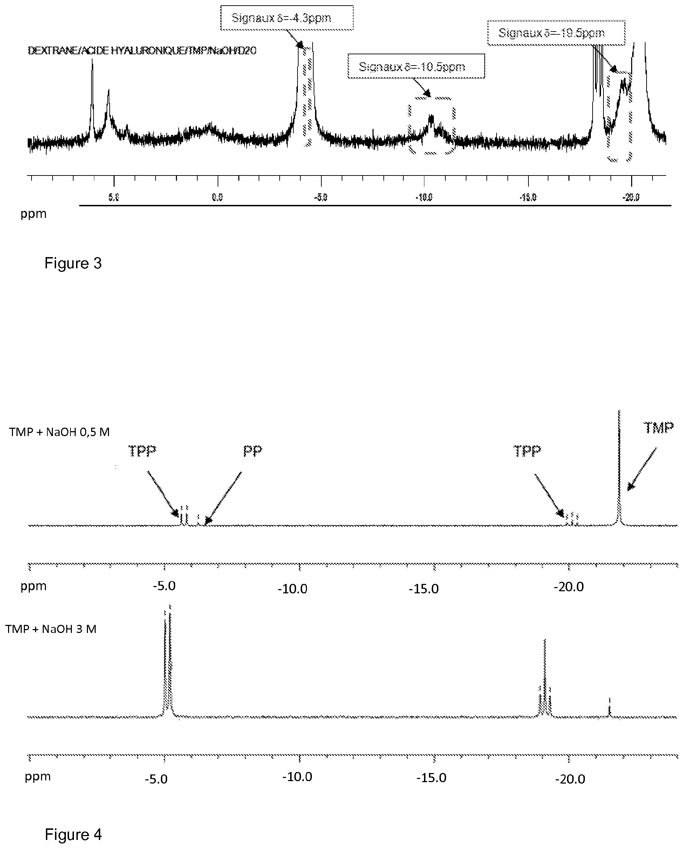

Le spectre RMN ³¹P (Bruker 250MHz - Analyses RMN du phosphore ; mode : irradiation des protons ; fréquence : 101MHz) de l'hydrogel récupéré a également été analysé (solvant D₂O) et est représenté en figure 3. La présence de signaux caractéristiques à des déplacements δ=-4,3 ppm ; δ=-10,5 ppm et δ=-19,5 ppm mettent en évidence la formation d'esters phosphoriques.

### v) Détermination de la teneur en phosphate

Un dosage de phosphate a été réalisé à partir d'un hydrogel obtenu selon l'invention, déshydratée puis hydrolysé en milieu acide (HCl 4N) dans les conditions suivantes :

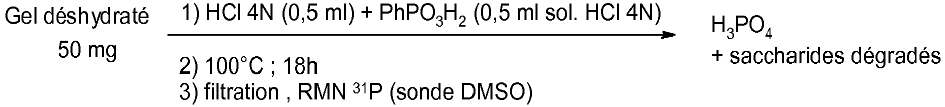

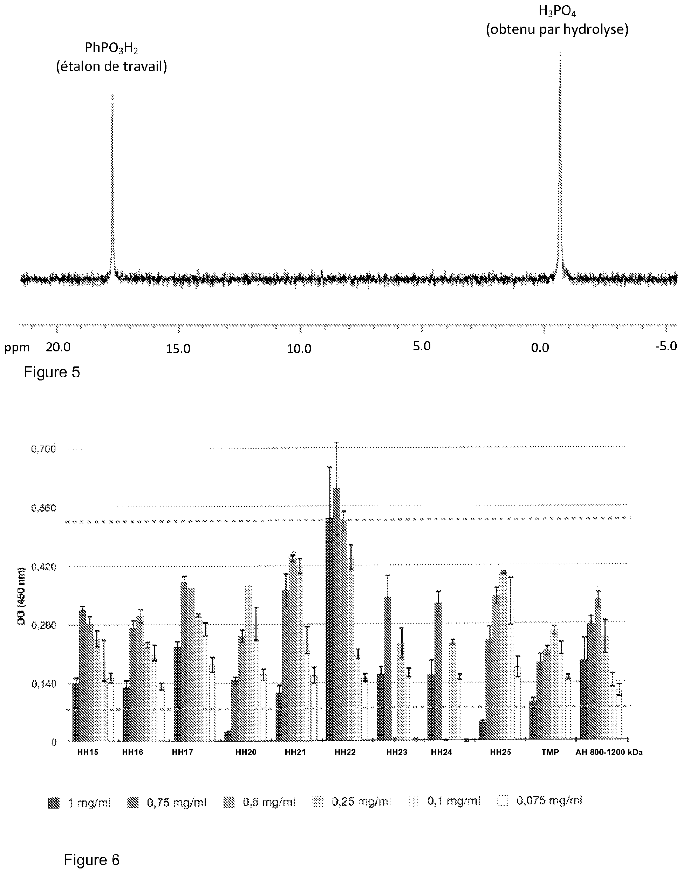

Les phosphates et polyphosphates liés sous forme d'esters aux motifs saccharidiques sont hydrolysés en milieu acide. Après traitement, ils se transforment indifféremment en motifs monomériques d'acide orthophosphorique. La RMN ³¹P donne un signal dont le déplacement caractéristique à -0.64 ppm est caractéristique de l'acide orthophosphorique. Ainsi, il est possible de déterminer la quantité de phosphate présent dans l'hydrolysat. L'acide phénylphosphonique PhPO₃H₂ est utilisé comme étalon (cf. figure 5).

Teneur en phosphates: 1,06 mEq/g (1 mEq/g correspond à 1 mmole d'acide phosphorique généré après hydrolyse par gramme de gel déshydraté analysé).

### Exemple 2 : Préparation d'un acide hyaluronique fonctionnalisé (HH) par le trimétaphosphate de sodium (TMP) : obtention d'un acide hyaluronique phosphorylé.

Cet exemple a pour finalité de préparer un acide hyaluronique fonctionnalisé pouvant être utilisé lors de l'étape d) du procédé selon l'invention.

0,20 grammes d'acide hyaluronique (Sigma Aldrich - n° CAS 9004-61-9 - 500K, 400-600 kDa) présentant une distribution de masse moléculaire dans l'intervalle 400 kDa - 600 kDa, ont été mis en solution dans 30 ml d'eau, puis 0,3 ml d'hydroxyde de sodium NaOH 0,1 M (n° CAS : 1310-73-2) ont été ajoutés à la solution. Le mélange a été maintenu sous agitation pendant 30 minutes à température ambiante.

0,09 grammes de trimétaphosphate de sodium (Sigma Aldrich - n° CAS 7785-84-4) ont été solubilisés dans 10 ml d'eau puis ajoutés dans la solution d'acide hyaluronique activé. Le mélange a été maintenu sous agitation pendant 3 heures à température ambiante.

Le milieu réactionnel a ensuite été neutralisé par une solution d'acide chlorhydrique 1M (n° CAS 7-647-01-0) pour atteindre une valeur de pH proche de 6 et précipité dans de l'éthanol à 97% (Sigma Aldrich - n° CAS 64-17-5).

Le précipitât a été solubilisé dans l'eau osmose et dialysé pendant 48 heures dans un boudin de dialyse. La solution est ensuite concentrée dans des cellules Amicon^{®} (Millipore) et lyophilisée.

### Test de prolifération cellulaire

Ce test vise à quantifier l'impact de la fonctionnalisation de l'acide hyaluronique vis-à-vis de la prolifération des fibroblastes de derme humain. La prolifération cellulaire a été évaluée par un test de type ELISA, basé sur la détection de l'incorporation du Bromodéoxyuridine (Kit BrdU ELISA - Roche). Le BrdU est un analogue des bases azotées de l'ADN qui va être incorporé à celui-ci au cours de la réplication de l'ADN pendant la division cellulaire. Il est ensuite possible de le détecter par un anticorps spécifique couplé à une enzyme qui permettra ensuite la réalisation d'un test colorimétrique et l'évaluation de la prolifération.

Par rapport à leur précurseur, les acides hyaluroniques fonctionnalisés présentent plusieurs comportements selon leur degré de fonctionnalisation et leur concentration (cf. figure 6 + Tableau 2 ci-après) :
- on n'a observé aucune amélioration de la prolifération, toutes concentrations confondues : (HH015, HH016, HH017, HH023 et HH024) ;
- on a observé le même niveau d'amélioration de la prolifération, mais à des concentrations optimales inférieures à celle d'AH 1200 : HH020 et HH025 (pic à 0,1 ou 0,25 mg/ml contre 0,5 ou 1 mg/ml pour AH 1200) ;
- on a observé une amélioration très nette de la prolifération : HH021 et HH022.

**Tableau 2 : Caractérisations chimiques des acides hyaluroniques fonctionnalisés**

| | | Phosphates totaux (après hydrolyse) |
|---|---|---|
| | ratio TMP/OH | Phosphate (mEq/g) |
| HH15 | 0,10 | 0,43 |
| HH16 | 0,10 | 0,44 |
| HH17 | 0,24 | 1,05 |
| HH20 | 0,02 | 0,08 |
| HH21 | 0,07 | 0,30 |
| HH22 | 0,15 | 0,60 |
| HH23 | 0,10 | 0,43 |
| HH24 | 0,10 | 0,42 |
| HH25 | 0,10 | 0,44 |

### Exemple 3 : Préparation d'un hydrogel co-réticulé à partir d'un acide hyaluronique fonctionnalisé (HH) et de dextrane (procédé selon l'invention).

*i)* *Activation des groupes hydroxyles du dextrane par une solution de NaOH 0,5* M 0,9 grammes de dextrane T100 (CAS n° 9004-54-0 ; Mw = 100 000 g/mol) ont été mis en solution dans 50 ml d'eau, puis 5 ml d'hydroxyde de sodium NaOH 0,5 M ont été ajoutés à la solution. Le mélange a été maintenu sous agitation pendant 30 minutes à température ambiante.
*ii) Ajout de l'acide hyaluronique fonctionnalisé (HH 22) et de l'agent réticulant* 1,02 grammes de trimétaphosphate de sodium et 0,2 grammes d'acide hyaluronique fonctionnalisé (HH 22) ont été ajoutés simultanément (sous la forme de poudres, de manière spatialement séparée) à la solution de l'étape a). Le mélange a été maintenu sous agitation à température ambiante jusqu'à gélification, puis le gel obtenu a été conservé pendant 5 heures à 25°C.
*iii) Lavage, purification et séchage de l'hydrogel*

L'hydrogel a été broyé sur un tamis en inox et récupéré dans une solution d'acétone.

Plusieurs lavages dans l'eau ont été effectués avec un suivi du pH, jusqu'à l'obtention d'un pH < 7 des eaux de lavage. Pour chaque étape de lavage réalisé, l'hydrogel a été filtré sur Büchner avec un filtre en acétate de cellulose ;
Un dernier lavage avec de l'acétone a été effectué, puis l'hydrogel a été séché à l'étuve sous vide à 60°C.

### iv) Cvtotoxicité

Afin d'évaluer la capacité des cellules à adhérer et à proliférer en présence des hydrogels à base de dextrane et d'acide hyaluronique fonctionnalisé, l'absence de cytotoxicité des hydrogels selon l'invention a été testée. Pour cela, le relargage de composés dans le milieu de culture a été évalué. Les hydrogels secs ont été placés dans des puits de plaques de culture 6 puits (6 puits par hydrogel) et ont été mis à gonfler dans 4 ml de milieu de culture :
- DMEM (DMEM = milieu Eagle modifié de Dulbecco - PAA), un milieu de culture cellulaire qui contient des acides aminés, des sels minéraux (KCI, MgSO4, NaCl, NaH2PO3), du glucose et des vitamines (acide folique, nicotinamide, riboflavine et vitamine B12)) + 2,5% SVF (sérum de veau foetal - PAA) pour trois des puits ;
- DMEM + 10% SVF pour 3 autres.

Les gels ont ensuite été laissés 72h à l'incubateur à 37°C. Les surnageants de culture ont ensuite été collectés et centrifugés à 1200 rpm pendant 5 minutes pour retirer tout fragment de gel des surnageants. 200 µl de chaque surnageant ont ensuite été ajoutés dans des puits de culture de plaque 96 puits ensemencés 4h auparavant par 5000 fibroblastes dermiques humains adultes (HDFa - Tebu Bio), afin que les HDFa aient eu le temps d'adhérer. Chaque surnageant a été testé sur trois puits indépendants. Les cellules ont été placées à l'incubateur à 37°C pendant 96h puis un test de viabilité au MTT est réalisé.

Ce test est basé sur la dégradation par les déshydrogénases mitochondriales du sel de tetrazolium en formazan, un composé violet insoluble s'accumulant dans la mitochondrie. Après incubation de 4h à 37°C des cellules en présences de 0,625 µg/ml de MTT, le milieu de culture a été retiré par renversement et les cristaux ont été dissous par ajout de 100µl d'un mélange volume à volume d'éthanol/DMSO. La lecture des résultats a été fait ensuite par spectrophotométrie, à une longueur d'onde de 570 nm.

Tous les hydrogels à base de dextrane et d'acide hyaluronique fonctionnalisé ne présentent aucun effet cytotoxique vis à vis des fibroblastes de derme humain.

### vi) Test de prolifération cellulaire

Deux conditions expérimentales ont été testées : (i) ensemencement des cellules sur une fine couche d'hydrogel ; (ii) mélange des cellules dans le gel au moment du gonflement directement dans le milieu de culture.

La viabilité cellulaire a été évaluée après 72h de culture des cellules en présence des hydrogels. Après l'ensemencement des cellules, les hydrogels ont été digérés par des enzymes hyaluronidases eucaryotes. Un test de viabilité d'exclusion au bleu de trypan sur les cellules récupérées a été réalisé. La colonisation des hydrogels par les cellules souches a été observée par microscopie optique et microscopie à balayage pour vérifier la répartition des cellules dans l'hydrogel.

### Exemple 4 : Test de relargage de facteur de croissance des fibroblastes (FGFb)

Cet exemple a pour finalité de démontrer la capacité des hydrogels selon l'invention à diffuser de manière contrôlée des facteurs de croissance au fur et à mesure de leur dégradation. Dans cet exemple, quatre hydrogels phosphorylés tests selon l'invention (DPD1, DPD2, DPD3, DPD4) et deux hydrogels témoin (DP1, DP2) ont été synthétisés. Les hydrogels témoin DP1 et DP2 sont formés uniquement de dextrane. La composition de ces hydrogels est présentée au Tableau 3 ci-après. Les compositions peuvent comprendre du dextrane T100 (100 000 g/mol) et/ou du dextrane T500 (500 000 g/mol).

**Tableau 3 : Composition des hydrogels synthétisés**

| Référence | Composition | % massique Dextrane | % massique de HA ou HH | Teneur en Phosphate (meq/g) | Ratio TMP/OH |
|---|---|---|---|---|---|
| DP1 (Témoin) | Dextrane T100 | T100 : 95 | 0 | 0,4 | 1,5 |
| | Dextrane T500 | T500 : 5 | | | |
| DP2 (Témoin) | Dextrane T100 | T100 : 90 | 0 | 0,5 | 1,5 |
| | Dextrane T500 | T500 :10 | | | |
| DPD1 | Dextrane T100 | T100 : 90 | 10 | 0,45 | 1,5 |
| | HA (1200 kDa) | | | | |
| DPD2 | Dextrane T100 | T100 : 90 | 10 | 0,47 | 1,5 |
| | HH (1200 kDa: 0,1 meq/g) | | | | |
| DPD3 | Dextrane T100 | T100 : 95 | 5 | 0,39 | 1,5 |
| | HH (800 kDa: 0,09 meq/g) | | | | |
| DPD4 | Dextrane T100 | T100 : 85 | 15 | 0,6 | 1,5 |
| | HH (300 kDa: 0,1 meq/g) | | | | |

Afin de vérifier l'affinité entre les hydrogels selon l'invention et les facteurs de croissance des fibroblastes, des expériences de relargage des facteurs de croissance ont été réalisées. Les hydrogels synthétisés (5 mg) ont été mis en contact avec le facteur de croissance (50 ng) au moment du gonflement des gels, puis sont placés dans une solution tampon de phosphate salin (PBS) à une température de 4°C. Après 12 h d'interaction, la solution tampon est remplacée par une solution aqueuse de chlorure de sodium NaCl 1M pendant 12 heures, puis par une solution de NaCl 1,5 M pendant 12 heures. Enfin, le liquide surnageant est récupéré pour déterminer par dosage le pourcentage de facteur de croissance non absorbé par l'hydrogel.

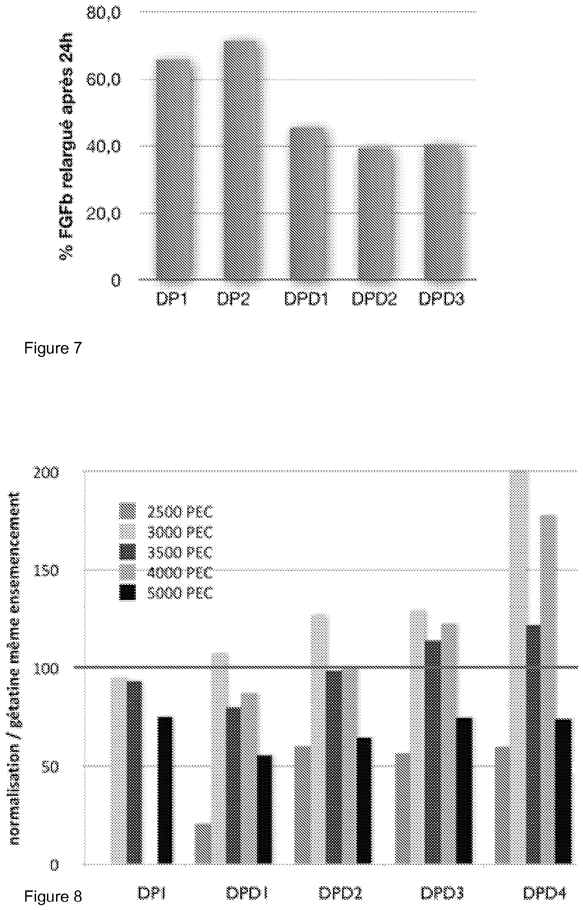

Sans vouloir être lié par une quelconque théorie, l'utilisation de la solution saline de chlorure de sodium, en augmentant la force ionique, permet de décrocher les facteurs de croissance des fibroblastes non absorbés de façon spécifique sur les hydrogels testés. Selon la composition de l'hydrogel, la capacité d'absorption des facteurs de croissance des fibroblastes est différente. Comme montré sur la figure 7, les hydrogels selon l'invention (DPD1, DPD2 et DPD3) retiennent 40% en masse de facteur de croissance après 24 heures de mise en contact dans une solution de NaCl contrairement aux hydrogels témoins (DP1 et DP2) constitués uniquement de dextrane, relarguant plus de 70% de facteurs de croissance après 24 heures.

Les hydrogels selon l'invention peuvent donc être utilisés pour un relargage contrôlé de facteur de croissance. Ainsi, une diffusion contrôlée est possible au cours du temps, ce qui permet de libérer les facteurs de croissance au fur et à mesure de la dégradation de l'hydrogel.

### Exemple 5 : test de prolifération de cellules

Ce test a pour finalité de tester la prolifération de cellules dans les hydrogels phosphorylés selon l'invention, en particulier sur des cellules souches, et plus particulièrement sur les progéniteurs endothéliaux circulant (appelés aussi PEC). Les progéniteurs endothéliaux circulant sont des cellules immatures, présentes dans le sang périphérique, où leur rôle physiologique est le maintien de l'intégrité vasculaire. Ils sont issus de la moelle osseuse, puis passent dans le sang, où ils constituent une population de cellules très rares. Ils s'intègrent à la formation de nouveaux vaisseaux, par exemple au cours du processus de revascularisation consécutif à une lésion vasculaire. En culture, ces cellules forment des colonies de cellules endothéliales, qui acquièrent rapidement un fort pouvoir de prolifération. La culture des cellules de progéniteurs endothéliaux circulants s'effectue sur un support type hydrogel qui va permettre leur survie et leur prolifération, et classiquement, la gélatine est utilisée comme support témoin de culture.

Dans cet exemples, différentes quantités initiales de cellules souches de type PEC ont été testées et après 48h de culture, une numération des PEC présentes a été réalisée. Les résultats sont exprimés en % massique et sont normalisés par rapport à la masse totale de l'hydrogel. D'après les résultats, et tel qu'illustré sur le tableau en figure 8, les hydrogels témoins DP1 et DP2 ne favorisent pas, voire ne permettent pas la prolifération cellulaire, et cela quel que soit le nombre de cellules initialement introduit. Les hydrogels selon l'invention, et plus particulièrement l'hydrogel DPD4, est un support de culture favorisant nettement la prolifération cellulaire puisque l'introduction initiale de 3 000 cellules permet d'augmenter de 100% la prolifération cellulaire en plus par rapport à l'hydrogel (cf. résultat en figure 8).

## Revendications

1. Hydrogel phosphorylé co-réticulé de polysaccharides, à base de (i) dextrane et (ii) d'acide hyaluronique et/ou d'un de ses sels, éventuellement fonctionnalisé, dans lequel hydrogel ledit dextrane et ledit acide hyaluronique (et/ou son sel, éventuellement fonctionnalisé) sont liés entre eux par des liaisons de covalence phosphodiesters et/ou polyphosphodiesters.

2. Hydrogel selon la revendication 1, dans lequel l'acide hyaluronique ou son sel est un acide hyaluronique (et/ou son sel) fonctionnalisé par le trimétaphosphate de sodium.

3. Hydrogel selon la revendication 2, **caractérisé en ce que** le taux de concentration en phosphate de l'acide hyaluronique (et/ou de son sel) fonctionnalisé par le trimétaphosphate de sodium est compris entre 0,01 et 4 mEq/g, plus préférentiellement entre 0,1 et 2 mEq/g.

4. Hydrogel selon l'une quelconque des revendications 1 à 3, **caractérisé en ce que** le taux de concentration en phosphate dudit hydrogel est compris entre 0,1 et 3 mEq/g, plus préférentiellement entre 0,1 et 2 mEq/g.

5. Procédé de préparation d'un hydrogel phosphorylé co-réticulé de polysaccharides, à partir de dextrane et d'acide hyaluronique et/ou un de ses sels, éventuellement fonctionnalisé, dans lequel procédé :
a) on approvisionne une solution de dextrane ;
b) on active au moins un groupe hydroxyle du dextrane en ajoutant une solution d'hydroxyde alcalin, par exemple de l'hydroxyde de sodium, à ladite solution de dextrane pour obtenir une solution de dextrane activé, la concentration en hydroxyde alcalin ajouté à l'étape b) étant comprise entre 0,5 M et 5 M, de préférence entre 0,5 M et 1 M ;
c) on ajoute à ladite solution de dextrane activé du trimétaphosphate de sodium ;
d) on ajoute à la solution obtenue à l'étape c) de l'acide hyaluronique et/ou un de ses sels, éventuellement fonctionnalisé ;
lesdites étapes a) à d) dudit procédé étant réalisées à une température comprise entre 18 et 25°C.

6. Procédé selon la revendication 5, **caractérisé en ce que** la concentration en trimétaphosphate dans le mélange réactionnel est comprise entre 0,26 M et 1 M, et le ratio [trimétaphosphate]/[OH] est compris entre 0,1 et 0,4.

7. Procédé selon la revendication 5 ou 6, **caractérisé en ce que** les étapes c) et d) sont réalisées concomitamment, le trimétaphosphate de sodium et l'acide hyaluronique et/ou un de ses sels, éventuellement fonctionnalisé, étant ajoutés concomitamment dans la solution obtenue à l'étape b) dudit procédé.

8. Procédé selon l'une quelconque des revendications 5 à 7, **caractérisé en ce que** l'acide hyaluronique et/ou un de ses sels ajouté à l'étape d) du procédé est un acide hyaluronique fonctionnalisé par le trimétaphosphate de sodium.

9. Procédé selon l'une quelconque des revendications 5 à 8, **caractérisé en ce que** ledit acide hyaluronique et/ou un de ses sels, éventuellement fonctionnalisé, est ajouté sous la forme d'une poudre.

10. Procédé selon l'une quelconque des revendications 5 à 9, **caractérisé en ce que** l'acide hyaluronique présente une masse moléculare comprise entre 10 kDa et 5 000 kDa, de préférence comprise entre 10 kDa et 1 200 kDa, et plus préférentiellement comprise entre 25 kDa et 1 200 kDa.

11. Procédé selon la revendication 10, **caractérisé en ce que** l'acide hyaluronique présente une masse moléculaire comprise entre 10 kDa et 50 kDa, ou entre 50 kDa et 300 kDa, ou supérieure à 800 kDa jusqu'à 5 000 KDa.

12. Hydrogel selon l'une quelconque des revendications 1 à 4 pour utilisation en régénération ossueuse, et en particulier pour l'ostéoconductivite et/ou l'ostéoinductivité.

13. Utilisation d'un hydrogel selon la revendication 1 ou 2 avec une teneur en phosphate d'au moins 0,01 mEq/g, de préréfence d'au moins 0,05 mEq/g, et encore plus préférentiellement d'au moins 0,1 mEq/g, en tant que support pour la culture cellulaire.

## Patentansprüche

1. Covernetztes phosphoryliertes Hydrogel von Polysacchariden auf der Basis von (i) Dextran und (ii) Hyaluronsäure und/oder eines ihrer Salze, gegebenenfalls funktionalisiert, wobei in dem Hydrogel das Dextran und die Hyaluronsäure (und/oder ihr Salz, gegebenenfalls funktionalisiert) durch kovalente Phosphodiester- und/oder Polyphosphodiester-Bindungen miteinander verbunden sind.

2. Hydrogel nach Anspruch 1, wobei die Hyaluronsäure oder ihr Salz eine Hyaluronsäure (und/oder ihr Salz) ist, die (bzw. das) durch Natriumtrimetaphosphat funktionalisiert ist.

3. Hydrogel nach Anspruch 2, **dadurch gekennzeichnet, dass** der Konzentrationsgrad von Phosphat der Hyaluronsäure (und/oder ihres Salzes), die (bzw. das) durch Natriumtrimetaphosphat funktionalisiert ist, zwischen 0,01 und 4 mÄq/g, mehr bevorzugt zwischen 0,1 und 2 mÄq/g liegt.

4. Hydrogel nach einem der Ansprüche 1 bis 3, **dadurch gekennzeichnet, dass** der Konzentrationsgrad von Phosphat des Hydrogels zwischen 0,1 und 3 mÄq/g, mehr bevorzugt zwischen 0,1 und 2 mÄq/g liegt.

5. Verfahren zur Herstellung eines covernetzten phosphorylierten Hydrogels von Polysacchariden aus Dextran und Hyaluronsäure und/oder einem ihrer Salze, gegebenenfalls funktionalisiert, wobei in dem Verfahren:
a) eine Dextranlösung bereitgestellt wird;
b) mindestens eine Hydroxylgruppe des Dextrans aktiviert wird, indem eine Lösung von alkalischem Hydroxid, beispielsweise Natriumhydroxid, zu der Dextranlösung zugegeben wird, um eine aktivierte Dextranlösung zu erhalten, wobei die Konzentration von alkalischem Hydroxid, das im Schritt b) zugegeben wird, zwischen 0,5 M und 5 M, vorzugsweise zwischen 0,5 M und 1 M liegt;
c) Natriumtrimetaphosphat zu der aktivierten Dextranlösung zugegeben wird;
d) Hyaluronsäure und/oder eines ihrer Salze, gegebenenfalls funktionalisiert, zu der im Schritt c) erhaltenen Lösung zugegeben wird;
wobei die Schritte a) bis d) des Verfahrens bei einer Temperatur durchgeführt werden, die zwischen 18 und 25 °C liegt.

6. Verfahren nach Anspruch 5, **dadurch gekennzeichnet, dass** die Konzentration von Trimetaphosphat in dem Reaktionsgemisch zwischen 0,26 M und 1 M liegt und das [Trimetaphosphat]/[OH]-Verhältnis zwischen 0,1 und 0,4 liegt.

7. Verfahren nach Anspruch 5 oder 6, **dadurch gekennzeichnet, dass** die Schritte c) und d) gleichzeitig durchgeführt werden, wobei das Natriumtrimetaphosphat und die Hyaluronsäure und/oder eines ihrer Salze, gegebenenfalls funktionalisiert, gleichzeitig in die im Schritt b) des Verfahrens erhaltene Lösung zugegeben werden.

8. Verfahren nach einem der Ansprüche 5 bis 7, **dadurch gekennzeichnet, dass** die Hyaluronsäure und/oder eines ihrer Salze, die bzw. das im Schritt d) des Verfahrens zugegeben wird, eine Hyaluronsäure ist, die durch Natriumtrimetaphosphat funktionalisiert ist.

9. Verfahren nach einem der Ansprüche 5 bis 8, **dadurch gekennzeichnet, dass** die Hyaluronsäure und/oder eines ihrer Salze, gegebenenfalls funktionalisiert, in der Form eines Pulvers zugegeben wird.

10. Verfahren nach einem der Ansprüche 5 bis 9, **dadurch gekennzeichnet, dass** die Hyaluronsäure eine Molmasse aufweist, die zwischen 10 kDa und 5000 kDa liegt, vorzugsweise zwischen 10 kDa und 1200 kDa liegt und besonders bevorzugt zwischen 25 kDa und 1200 kDa liegt.

11. Verfahren nach Anspruch 10, **dadurch gekennzeichnet, dass** die Hyaluronsäure eine Molmasse aufweist, die zwischen 10 kDa und 50 kDa oder zwischen 50 kDa und 300 kDa oder mehr als 800 kDa bis zu 5000 kDa liegt.

12. Hydrogel nach einem der Ansprüche 1 bis 4 zur Verwendung bei der Knochenregeneration und insbesondere zur Osteokonduktivität und/oder Osteoinduktivität.

13. Verwendung eines Hydrogels nach Anspruch 1 oder 2 mit einem Gehalt von Phosphat von mindestens 0,01 mÄq/g, vorzugsweise mindestens 0,05 mÄq/g und noch mehr bevorzugt mindestens 0,1 mÄq/g als Träger für eine Zellkultur.

## Claims

1. Co-cross-linked phosphorylated polysaccharide hydrogel, based on (i) dextran and (ii) hyaluronic acid and/or one of its salts, optionally functionalized, in which hydrogel said dextran and said hyaluronic acid (and/or its salt, optionally functionalized) are bound together by phosphodiester and/or polyphosphodiester covalent bonds.

2. Hydrogel according to claim 1, wherein the hyaluronic acid or its salt is a hyaluronic acid (and/or its salt) functionalized by sodium trimetaphosphate.

3. Hydrogel according to claim 2, **characterized in that** the phosphate concentration level of the hyaluronic acid (and/or its salt) functionalized by sodium trimetaphosphate is between 0.01 and 4 mEq/g, more preferably between 0.1 and 2 mEq/g.

4. Hydrogel according to any one of claims 1 to 3, **characterized in that** the phosphate concentration level of said hydrogel is between 0.1 and 3 mEq/g, more preferably between 0.1 and 2 mEq/g.

5. Process for preparing a co-cross-linked phosphorylated polysaccharide hydrogel, based on dextran and hyaluronic acid and/or one of its salts, optionally functionalized, in which process:
a) a dextran solution is provided;
b) at least one hydroxyl group of the dextran is activated by adding an alkaline hydroxide solution, for example sodium hydroxide, to said dextran solution to obtain an activated dextran solution, the concentration of alkaline hydroxide added in step b) being between 0.5 M and 5 M, preferably between 0.5 M and 1 M;
c) sodium trimetaphosphate is added to said activated dextran solution;
d) hyaluronic acid and/or one of its salts, optionally functionalized, is added to the solution obtained in step c) ;
said steps a) through d) of said process being performed at a temperature of between 18 and 25°C.

6. Process according to claim 5, **characterized in that** the trimetaphosphate concentration in the reaction mixture is between 0.26 M and 1 M, and the ratio [trimetaphosphate]/[OH] is between 0.1 and 0.4.

7. Process according to claim 5 or 6, **characterized in that** steps c) and d) are performed concomitantly, the sodium trimetaphosphate and hyaluronic acid and/or one of its salts, optionally functionalized, being added concomitantly to the solution obtained in step b) of said process.

8. Process according to any one of claims 5 to 7, **characterized in that** the hyaluronic acid and/or one of its salts added in step d) of the process is a hyaluronic acid functionalized by sodium trimetaphosphate.

9. Process according to any one of claims 5 to 8, **characterized in that** said hyaluronic acid and/or one of its salts, optionally functionalized, is added in the form of a powder.

10. Process according to any one of claims 5 to 9, **characterized in that** the hyaluronic acid has a molecular mass of between 10 kDa and 5000 kDa, preferably between 10 kDa and 1200 kDa, and more preferably between 25 kDa and 1200 kDa.

11. Process according to claim 10, **characterized in that** the hyaluronic acid has a molecular mass of between 10 kDa and 50 kDa, or between 50 kDa and 300 kDa, or above 800 kDa and up to 5000 kDa.

12. Hydrogel according to any one of claims 1 to 4 for use for bone regeneration, and in particular for osteoconductivity and/or osteoinductivity.

13. Use of a hydrogel according to claim 1 or 2 with a phosphate content of at least 0.01 mEq/g, preferably at least 0.05 mEq/g, and even more preferably at least 0.01 mEq/g, as a medium for cell culture.
